# EUROPEAN PATENT APPLICATION

(11) **EP 4 813 319 A1**
(43) Date of publication of application: **30.09.2026**
(21) Application number: 24905515.3
(22) Date of filing: 19.07.2024
(51) Int. Cl.: A61B 17/128

(54) **CLIP APPLIER WORKING HEAD WITH LATERALLY-SLIDING REPLACEABLE CLIP CARTRIDGE AND CLIP APPLIER**

(30) Priority: 18.12.2023 CN 202311739831
(71) Applicant: Innolcon Medical Technology (Suzhou) Co., Ltd., Suzhou, Jiangsu 215000 (CN)
(72) Inventor: JI, Jianping, Suzhou, Jiangsu 215000 (CN); CHAI, Zhaoqiang, Suzhou, Jiangsu 215000 (CN); CHANG, Dingyao, Suzhou, Jiangsu 215000 (CN); ZHONG, Pengfei, Suzhou, Jiangsu 215000 (CN); LUO, Wei, Suzhou, Jiangsu 215000 (CN)
(74) Representative: Hoefer & Partner Patentanwälte mbB
(86) International application number: PCT/CN2024/106566
(87) International publication number: WO 2025/130017

(57) **Abstract**

Disclosed are a clip applier working head with a laterally-sliding replaceable clip cartridge and a clip applier, comprising a connector housing mated with a main body of the clip applier. The connector housing is driven by a driving element located at the distal end of the main body to produce corresponding actions. The distal end of the connector housing is provided with a retaining unit, and scissors-type hinged jaws are pivotally arranged on the retaining unit. The proximal end of the connector housing comprises an accommodating space extending laterally therethrough. The accommodating space is configured to accommodate a laterally-sliding insertable clip cartridge. Ligating clips are arranged sequentially from distal to proximal within the clip cartridge. The distal end of a push rod located within the main body abuts the most proximal ligating clip and sequentially pushes the ligating clips from the distal end of the clip cartridge into the retaining unit. The ligating clip located in the retaining unit is pushed out and fixedly placed in the jaws which are in an open state.

## Description

The present disclosure claims priority to Chinese Patent Application No. 202311739831.6 filed with the China National Intellectual Property Administration on December 18, 2023, and the entire contents of which are incorporated herein by reference.

### FIELD OF THE INVENTION

The present disclosure relates to the technical field of surgical robots, and in particular to a clip applier working head with a laterally-sliding replaceable clip cartridge and a clip applier.

### BACKGROUND OF THE INVENTION

The benefits of minimally invasive surgery are well known, and compared with traditional open-incision surgery, they include less trauma to the patient, less blood loss, and faster recovery time. During minimally invasive surgery, when a patient is bleeding, a clip applier is required to clamp the blood vessel (major artery) to prevent blood from flowing out.

A clip applier is a relatively commonly used surgical instrument, used to perform clamping operations on ligating clips (hemolok) during surgery, and is mostly used to clamp blood vessels for the purpose of hemostasis. For this reason, it is sometimes called a hemostatic forceps. The principle of the clip applier is the same as that of a clip, except that it has been sterilized. Clip appliers come in large, small, toothed, toothless, straight, and curved types. Different types of clip appliers are selected according to different operating sites.

Most existing clip appliers are single-tube single-shot clip appliers. In particular, clip appliers that are inserted into the abdominal cavity need to be loaded with ligating clips multiple times, which results in relatively low surgical efficiency. It would be very regrettable to miss the best ligation time, so it is very necessary to make the loading more convenient. When performing human endoscopic surgery, although a single-shot clip applier can accomplish the clip-applying function, and the clip applier can be used multiple times after sterilization, the instrument needs to be passed multiple times between the doctor and the nurse during the operation. That is, after the surgeon completes a ligation, the clip applier needs to be taken out from the human body and passed to a nearby nurse to reload the clip applier, and then passed back to the surgeon, who then inserts the loaded clip applier into the human body to perform the clip-applying task, and such operations have to be repeated multiple times. Using this kind of single-shot clip applier to complete the operation of ligating a blood vessel with a ligating clip takes a long time, and may even cause shock or even death due to excessive blood loss, thereby increasing the surgical risk. Therefore, in clinical practice, doctors and patients tend to use continuous-firing clip appliers, which can reduce surgical time and lower surgical risk.

In view of this, Chinese Patents 201980051943.8 and 202111376487.X disclose different forms of continuous-firing clip appliers, in which a plurality of ligating clips are pre-loaded into the ligating clip accommodating cavity, and a driving rod causes the feeding rod to slide relative to the magazine housing, thereby extending into the ligating clip accommodating cavity to push the ligating clips, so that the ligating clips are pushed out in sequence, thereby achieving continuous clip application. Since the ligating clips are arranged sequentially in the ligating clip accommodating cavity, the ligating clip accommodating cavity becomes excessively long, and the overall length of the clip applier also becomes longer. Chinese Patent 202222820091.6 discloses a clip applier with a replaceable clip cartridge assembly, which adopts a push-button installation manner, resulting in a relatively complex structure of the clip cartridge assembly.

### SUMMARY OF THE INVENTION

In one aspect, the present disclosure provides a clip applier working head with a laterally-sliding replaceable clip cartridge, comprising a connector housing mated with a main body of the clip applier, wherein the connector housing is driven by a driving element located at the distal end of the main body to produce corresponding actions, the distal end of the connector housing is provided with a retaining unit, scissors-type hinged jaws are pivotally arranged on the retaining unit, the proximal end of the connector housing includes an accommodating space extending laterally therethrough, the accommodating space is configured to accommodate a laterally-sliding insertable clip cartridge, ligating clips are arranged sequentially from distal to proximal in the clip cartridge, the distal end of a push rod located in the main body abuts the most proximal ligating clip and sequentially pushes the ligating clips from the distal end of the clip cartridge into the retaining unit, and the ligating clip located in the retaining unit is pushed out and fixedly placed in the jaws which are in an open state.

In another aspect, the present disclosure provides a clip applier, having a main body and the clip applier working head as described above located at the distal end of the main body.

The beneficial effects of the present disclosure are mainly reflected in the following aspects:
(1) The clip cartridge can be taken out and placed in a laterally-sliding manner, the clip cartridge has a simple structure, and convenience is improved; in actual operation, it is only necessary to be equipped with a sufficient number of clip cartridges;
(2) The arrangement manner of the ligating clips within the clip cartridge is simple, which facilitates assembly; and
(3) The retaining unit is of an integrated type with integrated functions, and the overall structure is more compact, ensuring that the overall diameter of the clip applier working head is reduced, making it suitable for surgical operations in narrow spaces.

### BRIEF DESCRIPTION OF THE DRAWINGS

The technical solutions of the present disclosure will be further described below with reference to the accompanying drawings:
FIG. 1 is a perspective view of some embodiments of the present disclosure;
FIG. 2 is a front view of some embodiments of the present disclosure;
FIG. 3 is a perspective view, in a second direction, of some embodiments of the present disclosure, in which the clip cartridge is taken out;
FIG. 4 is a perspective view, in a second direction, of some embodiments of the present disclosure, in which the clip cartridge is placed in and the connector housing is hidden;
FIG. 5 is a cross-sectional view taken along line A-A in FIG. 2;
FIG. 6 is a structural view of the retaining unit of some embodiments of the present disclosure;
FIG. 7 is a perspective structural view of some embodiments of the present disclosure in the course of operation; and
FIG. 8 is a front view of some embodiments of the present disclosure in the course of operation.

### DETAILED DESCRIPTION OF THE EMBODIMENTS

The present disclosure will be described in detail below with reference to the specific embodiments shown in the accompanying drawings. However, these embodiments are not limited by the present disclosure, and any structural, methodological, or functional transformations made by a person of ordinary skill in the art based on these embodiments shall fall within the protection scope of the present disclosure. In the description of the solutions, it should be noted that, with the operator as a reference, the direction close to the operator is the proximal end, and the direction away from the operator is the distal end.

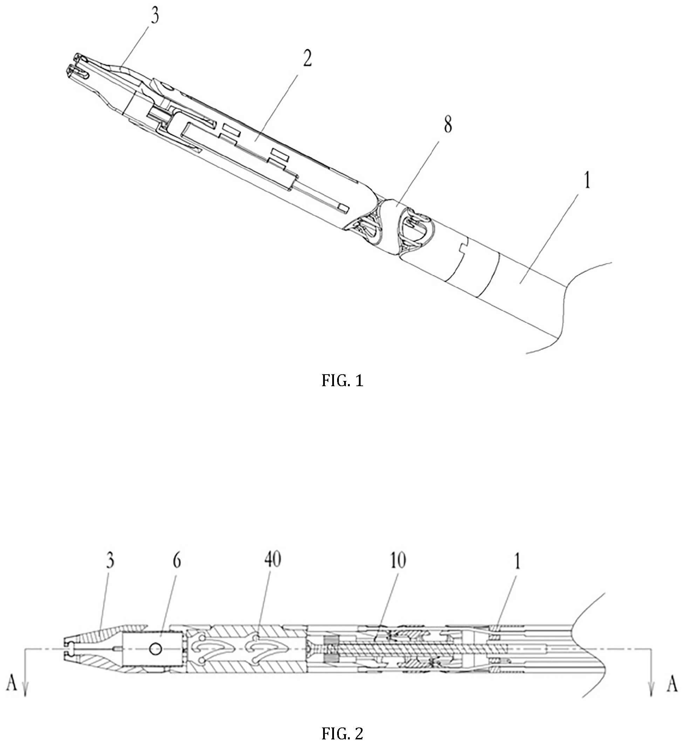

As shown in FIG. 1, some embodiments of the present disclosure provide a working head of a clip applier. As in the related art, the clip applier includes a main body 1 and a clip applier working head located at the distal end of the main body 1.

The clip applier working head includes a connector housing 2 mated with the main body 1 of the clip applier, and the connector housing 2 is driven by a driving element located at the distal end of the main body 1 to produce corresponding actions. For example, in some examples, the connector housing 2 can rotate about its own axis, or swing.

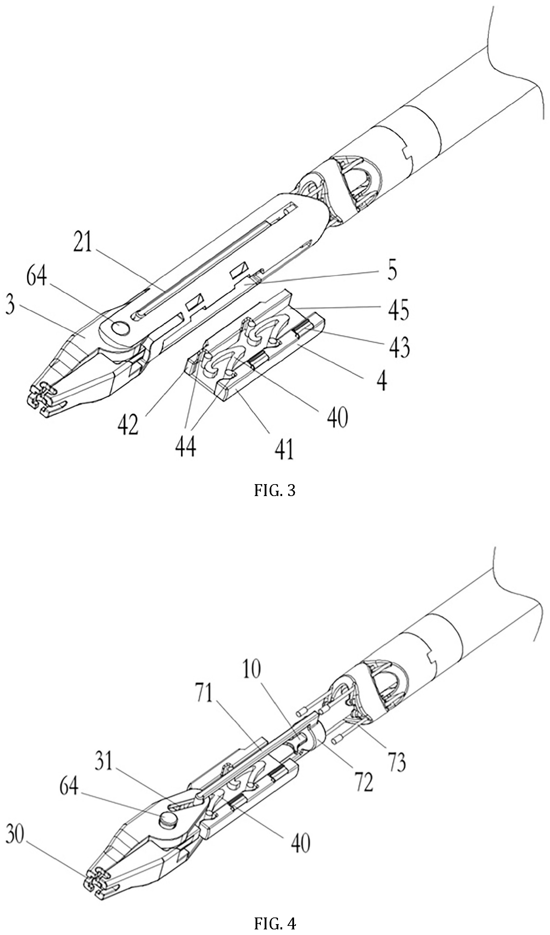

As can be seen in conjunction with FIG. 4, the driving element includes a set of pull cables 73, the distal end of each pull cable 73 is fixed to the connector housing 2, and the pull cables 73 are configured to drive the connector housing 2 to swing. In order to better achieve the swinging, an articulation ring 8 may be provided between the connector housing 2 and the main body 1.

One of the features of the present disclosure is that the distal end of the connector housing 2 is provided with a retaining unit 6, and scissors-type hinged jaws 3 are pivotally arranged on the retaining unit 6. In some embodiments of the present disclosure, one of the purposes of the retaining unit 6 is to provide a place where a ligating clip 40 is fixed therein, ready to be fired and pushed out.

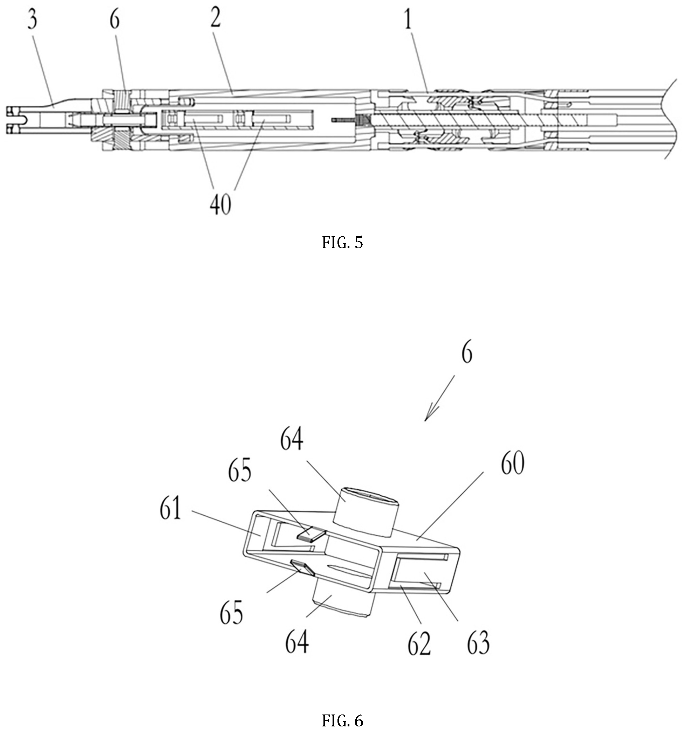

As shown in FIG. 6, the retaining unit 6 may have an integrated hollow-shell structure. The retaining unit 6 may include a housing 60 having a hollow structure, and the distal end and the proximal end of the housing 60 are each provided with an identical opening 61. The ligating clip 40 is pushed in from the proximal end, and subsequently pushed out from the distal end.

The left and right side surfaces of the housing 60 are each provided with a slot 62, so as to form an elastic piece 63 whose one end is integral with the proximal end of the housing 60. When the ligating clip 40 retained in the retaining unit 6 is located within the hollow shell of the retaining unit 6, since the ligating clip 40 itself is in a pre-compressed state, its two free ends have a tendency to spread outward, while the elastic piece 63 generates a corresponding counter-force, and the friction force produced thereby can effectively fix the ligating clip 40 within the hollow shell of the retaining unit 6.

Pivot posts 64 are coaxially provided on the outer sides of the upper and lower end surfaces of the housing 60, the purpose of which is to provide a pivot axis for the two separated structures of the jaws 3. The selective opening process of the jaws 3 will be described in detail below.

In some examples, through holes may be provided in the upper and lower end surfaces of the housing 60, and pivot shafts may be provided on the two separated structures of the jaws 3, with the pivot shafts being inserted into the through holes, thereby achieving the pivotal connection between the jaws 3 and the housing 60.

The distal end of the housing 60 may be provided with two guide pieces 65 arranged relatively obliquely, and the minimum distance between the two guide pieces 65 matches the thickness of the ligating clip 40.

In some examples, the minimum distance between the two guide pieces 65 and the thickness of the ligating clip 40 may be identical, close, or approximate.

This is because, due to unavoidable machining errors, it cannot be guaranteed that the clip cartridge 4 and the jaws 3 can be coaxially aligned. The guide pieces 65 play a guiding role, ensuring that the ligating clip 40 can stably enter the jaws 3.

The proximal end of the connector housing 2 includes an accommodating space 5 extending laterally therethrough. As shown in FIG. 3, the accommodating space 5 is a frame-shaped through slot, and the accommodating space is configured to accommodate a laterally-sliding insertable clip cartridge 4. The clip cartridge 4 can be pushed into the accommodating space 5 in a side-sliding manner.

The clip cartridge 4 has a plate-shaped structure, and has a plate body 45, wherein the plate body 45 can be configured to hold the ligating clips 40. One side of the plate body 45 has a first stopper 41 extending in the height direction, and the other side of the plate body 45 has a second stopper 42 extending in the height direction. The first stopper 41 and the second stopper 42 match the profiles of the two openings of the accommodating space 5 of the connector housing 2. When the clip cartridge 4 is pushed into the accommodating space 5 in a side-sliding manner, the clip cartridge 4 and the connector housing 2 form a nearly complete outer shape.

In some examples, the first stopper 41 may be provided with a resilient latch 43. The resilient latch 43 may be configured to engage with the connector housing 2.

In some examples, when the clip cartridge 4 is pushed into the accommodating space 5 in a side-sliding manner, the resilient latch 43 may engage with a mating structure on the connector housing 2, ensuring that the clip cartridge 4 does not slide out easily. When the clip cartridge needs to be replaced, pushing the second stopper 42 by hand can release the engagement, and the clip cartridge 4 can be smoothly taken out from the connector housing 2.

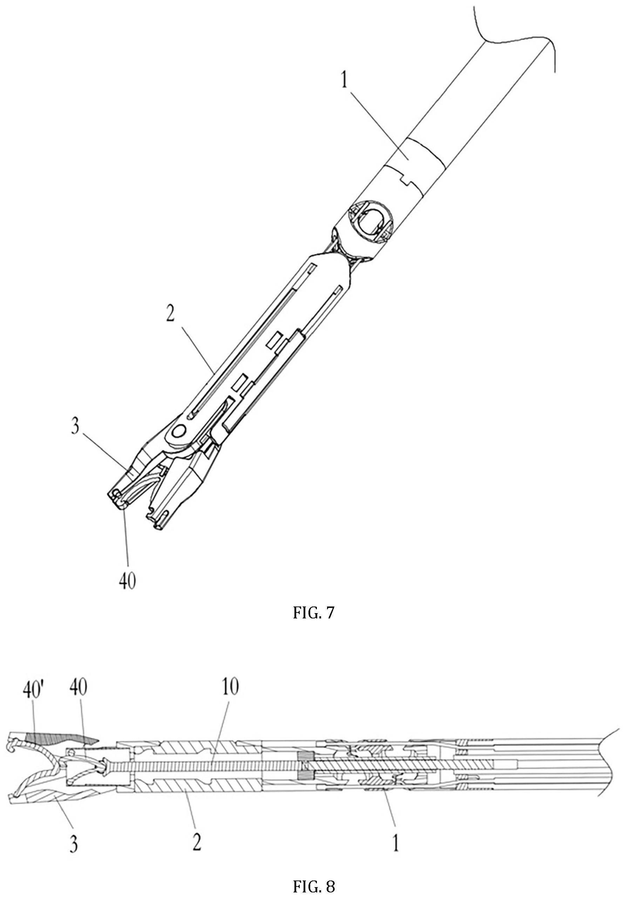

As shown in FIG. 4, a through hole may be provided in the connector housing 2, and the through hole may have a size consistent with that of the pivot post 64, so that the pivot post 64 can be inserted into the connector housing 2. The connector housing 2 has a slot 21, and the slot 21 may extend along the axial direction of the connector housing 2. The slot 21 may be configured to accommodate a pull rod 71. The pull rod 71 can slide within the slot 21 along the axial direction of the connector housing 2; the proximal end of the pull rod 71 is fixedly connected to a flexible shaft connector 72 of the driving element, and the distal end of the pull rod 71 is respectively connected to inclined slots 31 of the two separated structures of the jaws 3. When the pull rod 71 and the flexible shaft connector 72 are pulled and moved by the driving element within the main body, the jaws 3 can be driven to simultaneously rotate about the pivot posts 64 to present an open state (see FIG. 7), or a closed state.

As shown in FIGS. 2 to 5, ligating clips 40 are arranged sequentially from distal to proximal within the clip cartridge 4, and the first stopper 41 and the second stopper 42 correspondingly have a plurality of pairs of grooves 44, the grooves 44 being configured to engage the two ends of the ligating clip 40. In the initial state, the ligating clips 40 are accommodated in the clip cartridge 4 in a pre-compressed manner. Since the ligating clips 40 are arranged sequentially, their assembly during processing is ensured to be relatively simple. In addition, the clip-jamming problem caused by sideways tilting of the ligating clips does not occur.

In some examples, the distal end of the push rod 10 located within the main body 1 abuts the most proximal ligating clip 40, and the distal end of the push rod 10 is a Y-shaped pushing head. The push rod 10 is slidably arranged within the flexible shaft connector 72. As shown in FIGS. 7 and 8, after the clip cartridge 4 is pushed into the connector housing 2, the flexible shaft inside the main body is pushed toward the distal end, thereby driving the flexible shaft connector 72 to push forward, which in turn drives the pull rod 71 to push forward, and the jaws 3 can thereby present an open state. Subsequently, the push rod 10 pushes the ligating clip 40 toward the distal end. When the ligating clip 40 is separated from the engagement position, it is pushed from the distal end of the clip cartridge 4 into the retaining unit 6, and then the ligating clip 40 located in the retaining unit 6 is pushed out and fixed in the jaws 3. This is because the jaws 3 are also provided with engaging slots 30 for retaining the two ends of the ligating clip 40. The ligating clip 40' returns to the open state, while a new ligating clip 40 is retained in the retaining unit 6. After the ligating clip in the jaws 3 has completed the ligation, the push rod 10 then pushes the next ligating clip into the engaging slots of the jaws 3 of the clip applier head to complete the second ligation task.

It should be understood that, although the present specification is described in terms of embodiments, not every embodiment contains only a single independent technical solution. This manner of description in the specification is merely for the sake of clarity, and those skilled in the art should take the specification as a whole, and the technical solutions in the various embodiments may also be appropriately combined to form other embodiments that can be understood by those skilled in the art.

The series of detailed descriptions set forth above are merely specific descriptions of feasible embodiments of the present disclosure, and are not intended to limit the protection scope of the present disclosure. Any equivalent embodiments or modifications made without departing from the technical spirit of the present disclosure shall be included within the protection scope of the present disclosure.

## Claims

1. A clip applier working head with a laterally-sliding replaceable clip cartridge, comprising a connector housing (2) mated with a main body (1) of the clip applier, wherein the connector housing (2) is driven by a driving element located at a distal end of the main body (1) to produce corresponding actions, the distal end of the connector housing (2) is provided with a retaining unit (6), scissors-type hinged jaws (3) are pivotally arranged on the retaining unit (6), a proximal end of the connector housing (2) comprises an accommodating space (5) extending laterally therethrough, the accommodating space (5) is configured to accommodate a laterally-sliding insertable clip cartridge (4), ligating clips (40) are arranged sequentially from distal to proximal within the clip cartridge (4), the distal end of a push rod (10) located within the main body (1) abuts the most proximal ligating clip (40) and sequentially pushes the ligating clips (40) from the distal end of the clip cartridge (4) into the retaining unit (6), and the ligating clip (40) located in the retaining unit (6) is pushed out and fixedly placed in the jaws (3) which are in an open state.

2. The clip applier working head with a laterally-sliding replaceable clip cartridge according to claim 1, wherein the retaining unit (6) has an integrated hollow-shell structure, and comprises a housing (60) having a hollow structure, the distal end and the proximal end of the housing (60) are each provided with an identical opening (61), the left and right side surfaces of the housing (60) are each provided with a slot (62) so as to form an elastic piece (63) whose one end is integral with the proximal end of the housing (60), pivot posts (64) are coaxially provided on the outer sides of the upper and lower end surfaces of the housing (60), and the pivot posts (64) are configured to be pivotally connected to two separated structures of the jaws (3); the distal end of the housing (60) is further provided with two guide pieces (65) arranged relatively obliquely, and the minimum distance between the two guide pieces (65) matches the thickness of the ligating clip (40).

3. The clip applier working head with a laterally-sliding replaceable clip cartridge according to claim 2, wherein a through hole is provided in the connector housing (2), the size of the through hole matches that of the pivot post (64), so that the pivot post (64) can be inserted into the connector housing (2); the connector housing (2) has a slot (21), the slot (21) is configured to accommodate a pull rod (71) such that the pull rod (71) slides within the slot (21); the proximal end of the pull rod (71) is fixedly connected to a flexible shaft connector (72) of the driving element, and the distal end of the pull rod (71) is respectively connected to inclined slots (31) of the two separated structures of the jaws (3), so as to drive the two to simultaneously rotate about the pivot posts (64) to present an open state.

4. The clip applier working head with a laterally-sliding replaceable clip cartridge according to claim 3, wherein the distal end of the push rod (10) is a Y-shaped pushing head.

5. The clip applier working head with a laterally-sliding replaceable clip cartridge according to claim 3, wherein the push rod (10) is slidably arranged within the flexible shaft connector (72).

6. The clip applier working head with a laterally-sliding replaceable clip cartridge according to claim 1, wherein the clip cartridge (4) has a plate-shaped structure, and has a plate body (45), the plate body (45) is configured to hold ligating clips (40), one side of the plate body (45) has a first stopper (41) extending in the height direction, the other side of the plate body (45) has a second stopper (42) extending in the height direction, the first stopper (41) and the second stopper (42) match the profiles of two openings of the accommodating space (5) of the connector housing (2), the first stopper (41) is further provided with an resilient latch (43), and the resilient latch (43) is configured to engage with the connector housing (2).

7. The clip applier working head with a laterally-sliding replaceable clip cartridge according to claim 6, wherein the first stopper (41) and the second stopper (42) correspondingly have a plurality of pairs of grooves (44), and the grooves (44) are configured to engage two ends of the ligating clip (40).

8. The clip applier working head with a laterally-sliding replaceable clip cartridge according to claim 1, wherein the jaws (3) are provided with engaging slots (30).

9. A clip applier, having a main body (1), and the clip applier working head according to any one of claims 1-8 located at the distal end of the main body (1).

10. The clip applier according to claim 9, wherein the driving element comprises a set of pull cables (73), the distal end of each pull cable (73) is fixed to the connector housing (2), and the pull cables (73) are configured to drive the connector housing (2) to swing.
